# EUROPEAN PATENT SPECIFICATION

(11) **EP 1 088 561 B1**
(45) Date of publication and mention of the grant of the patent: **04.06.2003**
(21) Application number: 00117212.1
(22) Date of filing: 11.08.2000
(51) Int. Cl.: A61L 9/12

(54) **A device for the controllable transfer of a liquid and an apparatus for dispensing transferred liquids**
Gerät zur kontrollierbaren Übertragung einer Flüssigkeit und Anordnung zur Abgabe von übertragenen Flüssigkeiten
Dispositif pour le transfert contrôlable d'un liquide et appareil pour la distribution de liquides transférés

(30) Priority: 29.09.1999 EP 99810876
(43) Date of publication of application: 04.04.2001
(73) Proprietor: Givaudan SA, 1214 Vernier-Genève (CH)
(72) Inventor: Hart, Gerald Leslie, Surbiton, Surrey KT5 8BD (GB); Naish, Guy Edward, Narborough, Leicester LE9 5DD (GB)
(74) Representative: Patentanwälte Schaad, Balass, Menzl & Partner AG

(56) References cited:
- EP-A- 0 864 330
- FR-A- 2 774 597
- US-A- 4 889 286

## Description

The present invention relates to a device for the controllable transfer of a liquid according to claim 1 and an apparatus for dispensing transferred liquids with at least one of said devices according to claim 15.

### BACKGROUND OF THE INVENTION

In order to transfer liquids it is known to use capillary action which is dependent on the cohesion forces within the liquid and the adhesion forces of the liquid to a capillary medium comprising small channels for the transfer of the liquid. Such a capillary medium, being introduced through an opening into a container, can therefore transfer a liquid out of said container by means of capillary action. The transferred liquid may be used to distribute chemical substances to the ambient air in order to mask or generate an odour, to evoke a medical or organoleptic effect or to affect insects.

The US 5 114 625 discloses a fragrance dispenser with a fluid reservoir and a wick with a fluid receiving end disposed in the reservoir for drawing liquid therefrom into the wick and an portion of the wick exposed to an air flow provided by a fan.

In order to stop the transfer of liquid the capillary medium must be removed or covered. To control the amount of fluid evaporated from the wick, the device disclosed in US 5 114 625 comprises a wick cover that is movable between a substantially closed position covering the exposed portion of the wick and an open position where the wick is exposed to the flow of air.

Removing or covering the capillary medium is inconvenient and may cause further distribution of remainders of the liquid on the cover or the removed capillary medium. The use of two or more liquids increases the inconvenience accordingly.

The present invention is therefore based on the object of specifying a device, which allows an easily controllable transfer of a liquid through a capillary medium and an apparatus for dispensing transferred liquids with at least one of said devices.

### SUMMARY OF THE INVENTION

The above and other objects of the present invention are achieved by a device as specified in claim 1 and an apparatus as specified in claim 15.

The inventive device allows the controllable transfer of a liquid, such as a perfume, a pharmaceutical agent, a masking agent, an insecticide or an organoleptic substance, by a first capillary medium including a wick from a container to the outlet opening, preferably further to a second capillary medium such as a gauze. The first capillary medium is designed such that it allows entering of liquid at its bottom portion and is impermeable for liquid through surfaces other than those of the bottom portion, by comprising a sleeve around its lateral surfaces Preferably, the first capillary comprises a wick made of a porous or woven or non-woven natural or synthetic material, as this is cheap to manufacture. Further, the position of the bottom portion of the first capillary medium is fixed relative to the container, preferably by being rigid itself or stabilised in shape by stabilising means, e.g. said sleeve or a rigid coating. Therefore, its position relative to the container is maintained when the device is turned. By liquid entering only at the bottom portion of the capillary medium and the medium maintaining its location with respect to the container it is achieved that the transfer or transport of liquid can be controlled, in general can be switched on and off, by altering the spatial position of the device, e.g. turning it upside-down.

In another embodiment of the invention the first capillary medium is inserted into a holder comprising a fitting . being designed to embrace the capillary medium and to fit into an opening of the container. Said holder further comprises a preferably non-porous sleeve which further embraces the capillary medium at -least at a length which is chosen in such a way, that, in a first vertical position of the sleeve, liquid has access and in a second, inverse, vertical position of the sleeve the liquid has no access to the capillary medium.

The inventive device is therefore easily controllable by turning it from a first to a second position, e.g. turning it upside-down.

In a preferred embodiment of the device the end of the sleeve extends to a side-wall of the container in such a way that in a third, horizontal position of the container access of the liquid to the capillary medium is prevented as well. Preferably this embodiment is used for an apparatus comprising two or more inventive devices which in positions one and two are activated alternatively and which are both deactivated in the third position. A further advantage of this embodiment is, that two or more liquids can be diffused separately.

In a further embodiment the first capillary medium is connected to a second capillary medium such as a gauze or an extended portion of the first capillary medium, from which the transferred liquid is diffused preferably under the support of an the air flow generated by fan.

In a further embodiment the device comprises an annular container with one or more, preferably three, compartments respective reservoirs filled with different liquids which through a first capillary medium are guided to a second capillary medium which is embraced by the container. The annular container may be moulded in one piece which can be produced at low cost. The annular container may however also consist of segments which can be separated for example for the refill of the liquids and combined afterwards. Using an annular container also facilitates handling of the device and integration into an apparatus which automatically turns the device into positions in which transfer of selected liquids is initiated or terminated. For this purpose the outer surface of the device, which is hold in a bearing, may be in contact with a drive wheel.

In order to reduce costs for the assembly of the devices the holders for the first capillary medium can be integrated into the containers of the inventive devices.

The first and the second capillary medium may be made in one piece. This would be especially advantageous in cases where the container can be disassembled and reassembled, embracing the second capillary medium in the centre.

The inventive apparatus, which allows dispensing of transferred liquids, comprises at least one inventive device as described above being rotatably mounted on an axis of a stator between at least a first vertical and a second position, where no liquid enters the first capillary medium.

In a principal embodiment the apparatus comprises two inventive devices arranged towards each other along the same axis on a rotor with the second capillary medium oriented towards the centre of the rotor in such a way that in a first position of the rotor only one of the devices and in the second position the other one of the devices transfers the liquid. To increase the diversity of dispensed liquids three devices are located in the same plane perpendicular to the rotational (first) axis on an imaginary circle with 120° spacing between two devices. Further, an apparatus with four to six devices located equally distributed on an imaginary sphere is advantageous as it allows to switch on and off the transfer of a single liquid out of four to six different liquids. Generally, the shape of the device and the maximum amount of liquid contained are such that the permeable bottom part of the first capillary medium is in contact with the liquid when the device is in the "on-position" (first position), and not in contact with the liquid when moving the device from the "on-position" to any of the "off-positions" (second position), e.g. by turning the rotor by 90° or 120° with respect to the horizontal.

The apparatus is preferably equipped with a control unit comprising a sensor for detecting the position of the rotor. Said control unit being capable to actuate the air flow generator and/or to control the air flow depending on the position of the rotor.

In the second ("off") position the diffusion of the liquid is therefore immediately stopped since liquid is no longer transferred by the first capillary medium and no longer diffused at the outlet opening or from the second capillary medium.

In a further embodiment the apparatus can be fully automated.

### BRIEF DESCRIPTION OF THE DRAWINGS

Some of the objects and advantages of the present invention have been stated, others will appear when the following description is considered together with the accompanying drawings, in which:
- Fig. 1: shows a holder 10 and a first capillary medium 3, separated from each other;
- Fig. 2: shows the holder 10 with the installed capillary medium 3;
- Fig. 3: shows a container 20 with an opening 22 suitable for inserting the holder 10;
- Fig. 4: shows the container 20 with the holder 10 and the capillary medium 3 installed;
- Fig. 5: shows the container 20 of Fig. 4 turned upside down;
- Fig. 6: shows a modified container 201 comprising two reservoirs 204a, 204b;
- Fig. 7: shows the first capillary medium 3 connected to a second capillary medium 5;
- Fig. 8: shows the arrangement of Fig. 7 installed in a container 20 of a first and a second inventive device 1x respective 1y;
- Fig. 9: shows the two inventive devices 1x, 1y of Fig. 8 in horizontal alignment and a third, vertically aligned inventive device 1z transferring liquid through the capillary media 3 and 5;
- Fig. 10: shows an inventive apparatus with the devices 1x, 1y of Fig. 8 installed,
- Fig. 11: shows an inventive device 101 with an annular container 2000 comprising one reservoir;
- Fig. 12: shows an inventive device 102 with an annular container 2001 comprising three reservoirs;
- Fig. 13: shows the inventive device 102 of Fig. 12 cut along line A-A and
- Fig. 14: shows a segment 102a' of an annular container.

### DETAILED DESCRIPTION OF THE PREFERRED EMBODIMENTS

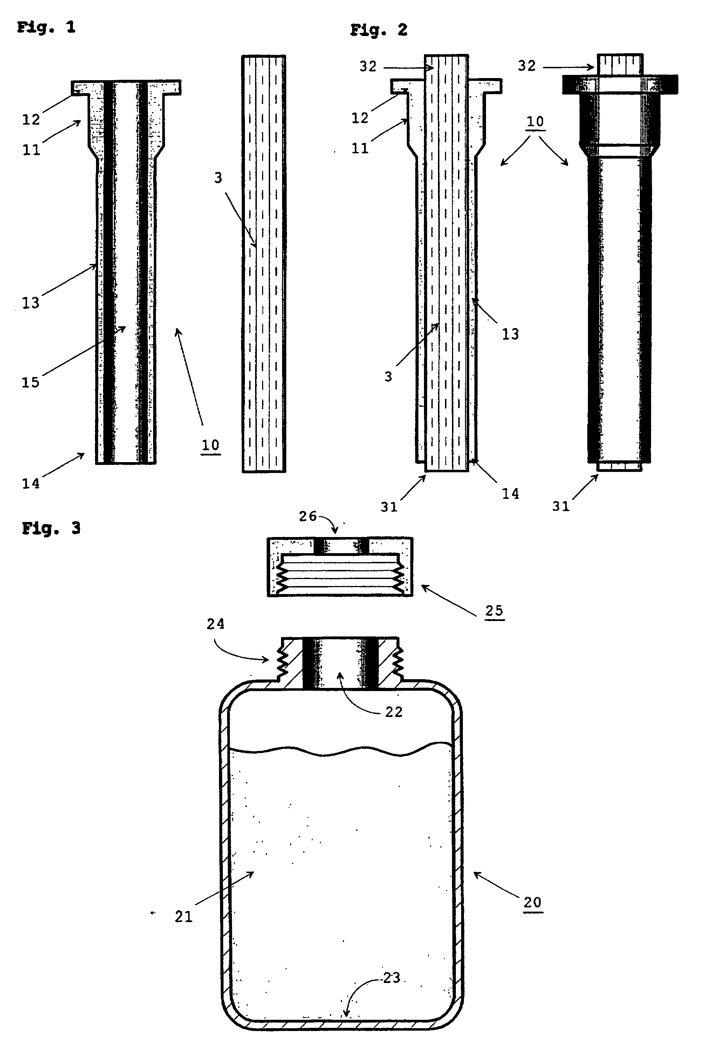

Fig. 1 shows a first capillary medium 3 and a holder 10 (in a sectional view) comprising a fitting 11 with a flange 12; said fitting 11 being connected to a sleeve 13. Fitting 11 and sleeve 13 build a tube with a channel 15 into which the capillary medium 3 can be inserted.

Fig. 2 shows the holder 10 with the installed capillary medium 3. The length of the capillary medium 3 is chosen in such a way that it is longer than fitting 11 and sleeve 13 combined. When installed, one end 31 of the capillary medium 3 will extend out of the sleeve 13 respective the end 14 of the sleeve 13 and the other end 32 will extend out of the fitting 11, which tightly seals the opening 22 after the installation.

Fig. 3 shows in a sectional view a container 20 with an opening 22 and a corresponding fitting 24 which is suitable to receive the fitting 11 of the holder 10 in such a way that the sleeve 13 is inserted into the container 20 and the flange 12 rests on the fitting 24 of the container 20 which is filled with a liquid 21 such as a perfume, a pharmaceutical agent, a masking agent, an insecticide or an organoleptic substance. Further shown is a cover 25 with an opening 26 through which the capillary medium 3 can be guided. The cover 25 can be screwed onto the fitting 24 of the container 20.

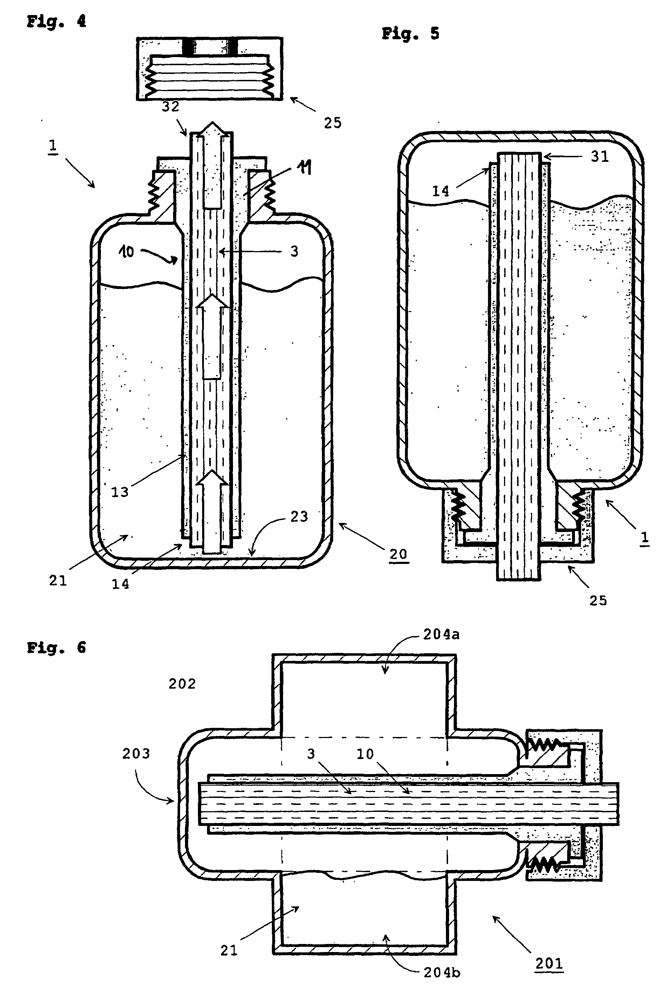

Fig. 4 shows the container 20 with the holder 10 and the capillary medium 3 installed. Due to the tight sitting of the fitting 11 in the opening 22 and the tight sitting of the capillary medium 3 in the channel 15 of the holder 10 the liquid 21 is only transferable through the opening 22 through the capillary medium 3 by capillary action. The liquid 21 can enter only at the end 31 of the capillary medium 3 respective at the end 14 of the sleeve 13 since the sleeve 13 shields the remaining part of the capillary medium 3, introduced into the container 20, against the liquid 21. The end 14 of the sleeve 13 of the installed holder 10 extends to a region close to the bottom 23 of the container 20.

Therefore, when turning the container 20 upside down, as shown in Fig. 5, the liquid 21 has no access to the capillary medium 3 which is shielded by the sleeve 13. Only in case that the container 20 were filled more than 95%, liquid could reach the capillary medium 3. However the capillary medium 3 and the sleeve 13 could also be extended to the bottom 23 of the container 20, comprising in there narrow channels for the liquid 21 to pass through to the capillary medium 3.

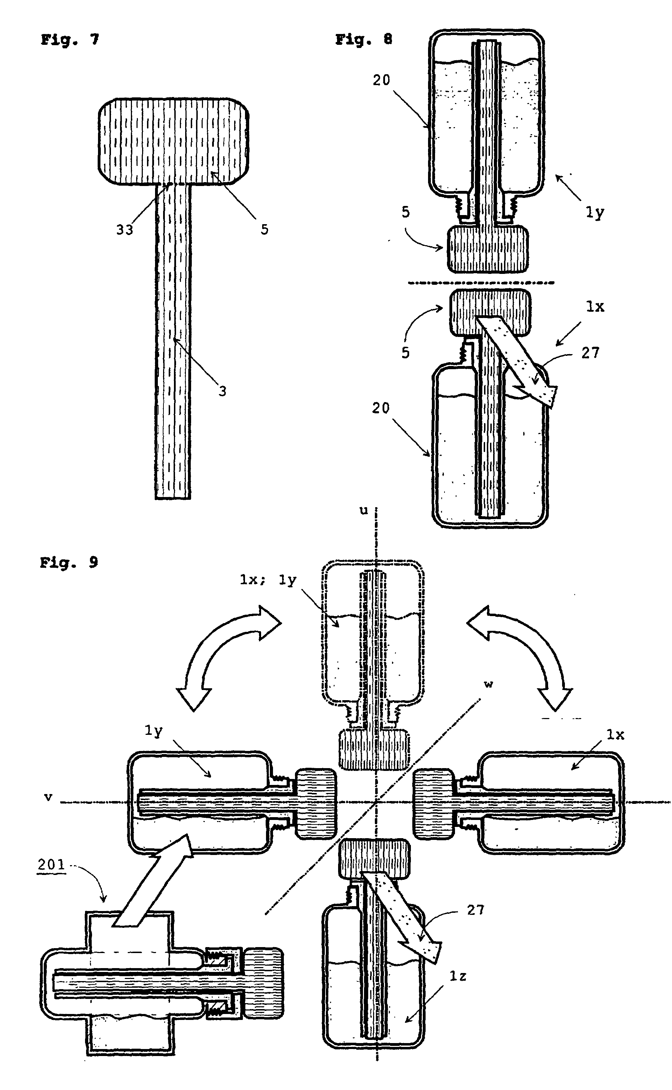

Fig. 6 shows a modified container 201 comprising two reservoirs 204a respective 204b at the side-wall 202, which receive the liquid 21 whenever the container 201 is turned into horizontal alignment. A clockwise rotation of the container 201 will cause the liquid 21 to enter reservoir 204b. A counter-clockwise rotation will cause the liquid 21 to enter reservoir 204a. The liquid 21 will therefore not be in contact with the capillary medium 3 in both horizontal orientations of the container 21. The reservoirs 204a, 204b may also be part of a cylindrical extension of the side-wall 202. The modified containers 201 are recommended for the use in arrangements comprising three or more inventive devices 1x, 1y, 1z. In the arrangement shown in Fig. 9 modified containers 201 could therefore advantageously be installed.

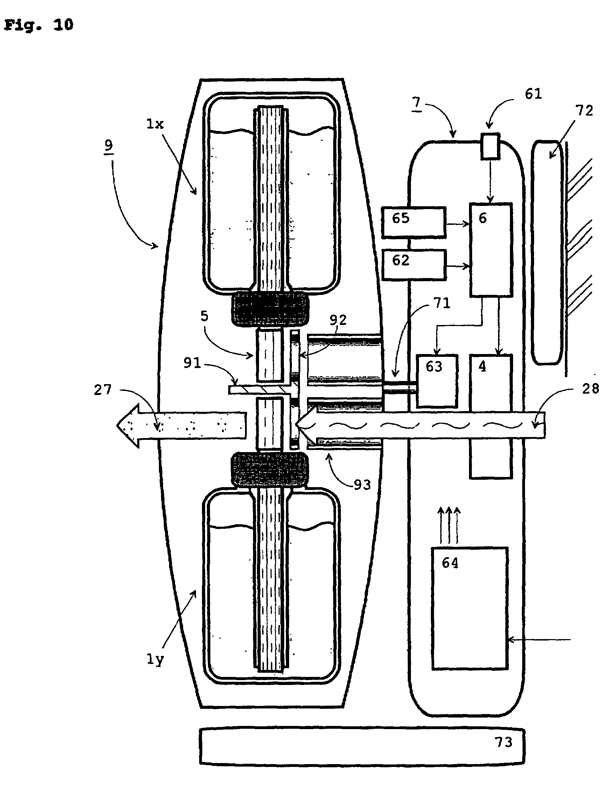

Fig. 7 shows the first capillary medium 3 connected to a second capillary medium 5 such as a gauze or an extended portion of the first capillary medium 3 which preferably consists of a porous synthetic material from which the transferred liquid 21 is exposed to the air or to the air flow 28 generated by an air flow generator 4 (see Fig. 10). From the first or second capillary medium 3; 5 the diffused liquid 27 (see Fig. 8, 9 or 10) is forwarded to the environment.

Fig. 8 shows the arrangement of Fig. 7 installed in a container 20 of a first and a second inventive device 1x respective 1y. The first device 1x is oriented as the device 1 shown in Fig. 4. As explained above in this orientation liquid 21 is transferred by the capillary medium 3. The second device 1y is oriented as the device 1 shown in Fig. 5 preventing the transfer of liquid 21.

Fig. 9 shows the two inventive devices 1x, 1y of Fig. 8 in horizontal alignment along an axis v and a third device 1z in vertical alignment along an axis u. The third device 1z is transferring liquid 21 through the capillary media 3 and 5 while no liquid is transferred by the horizontally aligned devices 1x, 1y, which contain only a small amount of liquid 21. Turning the arrangement around axis v by 90°, will cause transfer of liquid 21 to stop in device 1z as well. However, turning the arrangement around an axis w, which is perpendicular to the axis u and v, to the left or to the right by 90° will start transfer of liquid 21 in one of the devices 1x respective 1y while transfer of liquid 21 in the device 1z will cease.

Adding two inventive devices 1 aligned along axis w will extend the arrangement to five devices 1 which can be turned around axis v or w into corresponding positions where liquid 21 is transferred in the corresponding devices 1.

In order to allow the use of larger volumes of liquid 21 the devices 1x, 1y and 1z can be replaced by devices comprising a modified container 201 as shown in Fig. 6.

Of course the vertical and horizontal alignment need not be exact and can vary in a broad angle depending on the percentage of liquid 21 in the containers 20; 201 and the form (bent, straight) and length of the sleeve 13.

It is also possible to use a single inventive device 1 preferably in a housing which can be put on a surface in at least two positions in such away that in both positions (inverse and not inverse) the device 1 is at least approximately vertically aligned.

Fig. 10 shows an inventive apparatus for dispensing liquids 21 comprising a stator 7 which, on a axis 71, carries a rotor 9 with two axially aligned inventive devices 1x, 1y. Possible is also the installation of only one device 1x in the apparatus. In the present position only the lower device 1y is transferring liquid 21 through the first and second capillary medium 3; 5 from where it is forwarded to the environment by a flow of air 28 being generated by a fan 4, which is preferably located in the stator 7. The air flow 28 is preferably guided in channels 93 in the rotor 9 and in the stator 7 in order to maximise the efficiency. The second capillary media 5 of the first and the second device 1x, 1y may be framed and isolated from each other by a frame 91 comprising openings 92 for the air flow 28. The rotor 9 can now be turned between at least a first position, e.g. as shown in Fig. 10 and a position in which the rotor 9 is turned by 180°. In the first position, liquid 21 from the first device 1y is transferred and diffused, while no liquid 21 is transferred in the second device 1x. In the second position liquid 21 is transferred in the second device 1x while no liquid 21 is transferred in the first device 1y. The liquid 21 to be diffused can therefore easily be selected by turning the rotor 9. In case that no liquid 21 should be diffused the devices 1x and 1y are brought into a third position where they are horizontally aligned. In case that this third position is used in the apparatus, then preferably the containers 201 shown in Fig. 6 are installed.

The positions of the rotor 9 are preferably detected by a sensor 62 which is connected to a control unit 6 which is controlling the fan 4 and/or a motor 63 which in a preferred embodiment is used to turn the rotor between positions. In case that the rotor 9 reaches the third position driven by the motor 63 or manually the fan 4 is switched off by the control unit 6 since no liquid is transferred in this position. In a further embodiment, depending on the input from a sensor 65, which measures temperature and/or humidity, the control unit 6 adjusts the air flow generated by the fan 4 or actuates fan 4 and rotor 9 in a suitable way.

The electrical devices in the apparatus are connected to a power supply 64. In order to control the apparatus preferably one or more control buttons 61 (mains switch etc.) are installed. The apparatus may also be programmable and equipped with a timer. The apparatus may rest on a support 73 or may be installed on the wall by corresponding means 72.

Fig. 11 shows an inventive device 101 with an annular container 2000 comprising one reservoir which is filled with a liquid 21. The annular container 2000, which may also have a toroidal form, embraces a cylindrical capillary medium 5 such as a gauze or an extended portion of a first capillary medium which is embraced by a holder 10. As described above the holder 10 is inserted into the container 2000 in such a way that in the position shown in Fig. 11 liquid 21 is transferred through the first capillary medium. With a turn of the device 101 the holder 10 is lifted out of the liquid 21 thus preventing the transfer of the liquid 21.

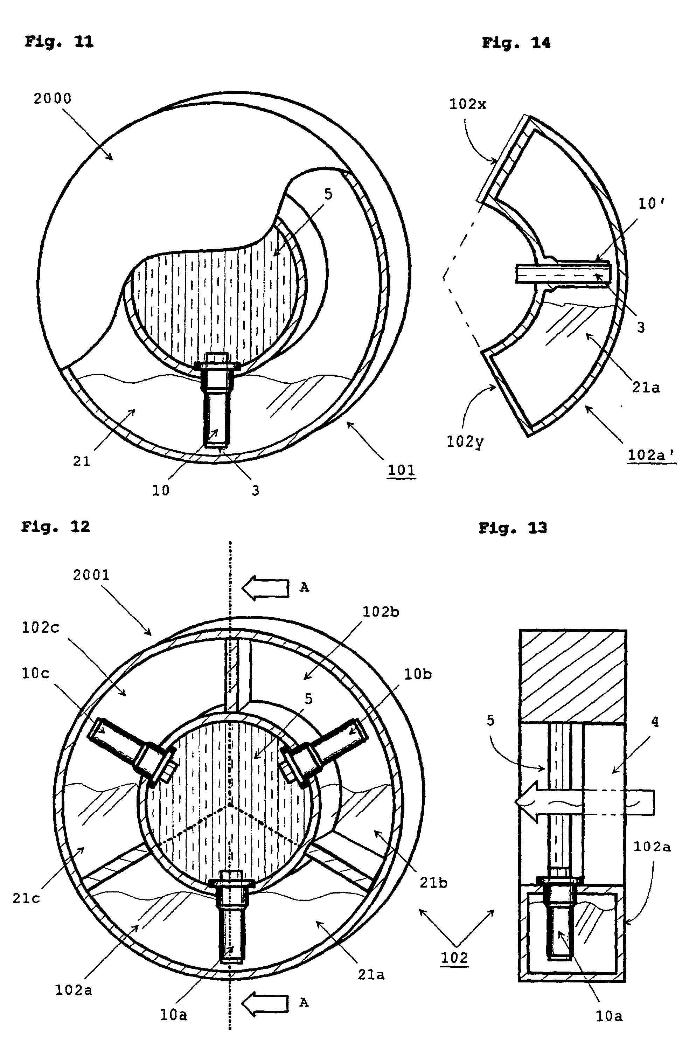

Fig. 12 shows an inventive device 102 with an annular container 2001 with three reservoirs 102a, 102b and 102c, each being filled with a liquid 21a, 21b, 21c and being equipped with a holder 10a, 10b, 10c for a first capillary medium. In the position shown in Fig. 12 the liquid 21a is transferred through the first capillary medium in the holder 10a to the second capillary medium 5. Transfer and distribution of the other liquids 21b or 21c can be initiated by turning the device 102 by approximately 120° to the left or to the right.

Fig. 13 shows the inventive device 102 of Fig. 12 cut along line A-A. It is shown that an air flow generator 4 is installed behind the second capillary medium 5 in such a way that the air flow generated is driven through the second capillary medium 5 thus distributing the transferred liquid 21a. The second capillary medium 5 can be formed of one piece, allowing the liquids to get mixed, or three pieces separated from each other, preventing the liquids to get mixed. The number of reservoirs 102 can be two, three or more.

The annular container 2001 of the inventive device 102 shown in Fig. 12 can be moulded in one piece. However the container 2001 can also be composed of three discrete segments 102a' as shown in Fig. 14. The shown segment 102a' preferably comprises means 102x, 102y for interconnecting all segments.

The holder 10' for the first capillary medium 3 is incorporated into the segment 102a'. Handling of this device is therefore facilitated, since liquid 21a can be refilled without removing the holder. Incorporation of the holder 10 can advantageously also be done in the containers 20, 201 shown in Fig. 1 to Fig. 10.

## Claims

1. A device (1) for the controllable transfer of a liquid (21) stored in a container (20; 201) through an outlet opening (22) of said container (20; 201) said device comprising container (20,201) an a first capillary medium (3) whose bottom portion extends into a region close to the bottom (23) of the container (20; 201), wherein liquid is transferable through the opening (22) due to capillary action, whereby the position of the bottom portion (31) of the first capillary medium (3) relative to the container (20; 201) is fixed, and
wherein the first capillary medium (3) includes a wick, **characterized in that**, the major part of the lateral surfaces of the wick being surrounded by a sleeve (13) impermeable to the liquid so that the surface of the wick (3) is permeable for liquid (21) at its bottom portion (31) and is impermeable for liquid (21) otherwise.

2. Device according to claim 1, wherein the layer (13) additionally serves to stabilise the position of the first capillary medium (3) relative to the container (20; 201).

3. Device according to claim 1, wherein the first capillary medium (3) comprises a number of capillaries.

4. Device according to one of the preceding claims, wherein the liquid transferred by the first capillary medium is exposed to air or to an air flow generated by an air flow generator (4) for evaporation.

5. A device (1) according to claim 1 wherein the container (20; 201) comprises a holder (10) for a capillary medium (3) which extends into a region close to the bottom (23) of the container (20; 201), said holder (10) comprising a fitting (11) being designed to embrace the capillary medium (3) and to fit into the opening (22) in such a way that liquid is only transferable through the opening (22) by the capillary medium (3), wherein the holder (10), adjacent to the fitting (11), further comprises said sleeve (13) which tightly embraces the capillary medium (3) up to or close to its end (31) so that the device (1) can be turned between at least a first position in which liquid (21) is transferred while contacting the end (31) of the capillary medium (3) and at least a second position in which the end (14) of the sleeve (13) extends out of the liquid (21) thereby preventing contact of the liquid (21) to the capillary medium (3).

6. Device (1) according to claim 5, wherein the capillary medium (3), which is extending out of the fitting (11), is connected to a second capillary medium (5) such as a gauze or an extended portion of the first capillary medium (3), from which the transferred liquid (21) is exposed to the air or to the air flow generated by an air flow generator (4) and evaporated.

7. Device (1) according to one of the preceding claims , wherein the container (201) is equipped with at least one reservoir (204a, 204b) which is suitable to receive the liquid (21) when the container (201) is turned into a third, approximately horizontal position.

8. Device (1) according to one of the preceding claims , wherein the opening (22) can be closed by a cover (25) comprising an opening (26) suitable for inserting the capillary medium (3).

9. Device (1) according to one of the claims 5 to 8, wherein the fitting (11) comprises a flange (12) which extends over the edges of the opening (22).

10. Device (1) according to one of the preceding claims , wherein the liquid (21) is a perfume, a pharmaceutical agent, a masking agent, an insecticide or an organoleptic substance.

11. Device (1) according to one of the claims 5 to 10, wherein the capillary medium (3) is a wick, the wick consisting of woven or non-woven fabric and/or a porous material.

12. Device (101; 102) according to one of the preceding claims, comprising an annular container (2000; 2001) with at least one reservoir (102a; 102b; 102c) being equipped with a holder (10; 10a; 10b; 10c) for the transfer of a liquid (21; 21a; 21b; 21c) through a first capillary medium (3) to a second capillary medium (5) which is embraced by the annular container (2000; 2001).

13. Device (102) according to claim 12 wherein the container (2001), which comprises two or more reservoirs (102a; 102b; 102c), is moulded in one piece or consists of interconnectable segments (102a') comprising a reservoir (102a).

14. Device (1; 101; 102) according to one of the claims 5 to 13, wherein the holder (10') is incorporated in the container (20; 201, 2000, 2001) respective the segment (102a').

15. Apparatus for dispensing at least one liquid (21), comprising at least one device (1x, 1y, 1z; 101; 102) according to one of the preceding claims for the controllable transfer of a liquid (21) through an outlet opening (22) of a container (20; 201; 2000; 2001) to the air, said device (1x, 1y, 1z; 101 102) being rotatably mounted between at least a first position in which liquid (21) is transferred and a second position in which transfer of liquid (21) is prevented.

16. Apparatus according to claim 15, wherein the liquid (21) is transferred to the air via a second capillary medium (5) connected to the first capillary medium (3).

17. Apparatus according to claim 15 or 16 with a rotor (9) which can be turned around at least a first axis (w) and with at least two devices (1x, 1y) installed in a plane perpendicular to the first axis (w) with 90°, 120° or 180° spacing of adjacent devices. in a first position of the rotor (9) liquid (21) is transferred only in a first device (1x) and that in a second position of the rotor (9) liquid (21) is only transferred in a second device (1y).

18. Apparatus according to claim 15, 16 or 17 with the rotor (9) being rotatable around the first axis (w) and a second axis (v) and with at least four devices being installed on the rotor (9) at approximately equal distances from each other on an imaginary sphere.

19. Apparatus according to one of the claims 15 to 18 with the outlet opening (22) and/or the second capillary medium (5) of the installed devices (1, 1x, 1y, 1z) being oriented towards the first axis (w) and/or the crossing point of the first and the second axis (v, w) .

20. Apparatus according to one of the claims 15 to 19 with a device (101) comprising an annular container (2000; 2001) with at least one reservoir (102a; 102b; 102c) being equipped with a holder (10; 10a; 10b; 10c) for the transfer of a liquid (21; 21a; 21b; 21c) through a first capillary medium (3) to a second capillary medium (5) which is embraced by the annular container (2000; 2001).

21. Apparatus according to one of the claims 15 to 20, comprising an air flow generator (4) such as a fan which is generating an air flow towards at least the outlet opening (22) or second capillary medium (5) of the device (1, 1x, 1y, 1z) whose first capillary medium (3) is transferring liquid (21).

22. Apparatus according to claim 21, further comprising a channel (93) to guide the air flow from the air flow generator (4) towards said outlet opening (22) or second capillary mediumn (3).

23. Apparatus according to one of the claims 15 to 22, with a control unit (6) comprising a switch (61) for actuating the air flow generator (4) and/or comprising a sensor (62) for detecting the position of the rotor (9) said control unit (6) being capable to actuate the air flow generator (4) and/or to control the air flow depending on the position of the rotor (9).

24. Apparatus according to claim 23, said control unit (6) being programmable and capable to control a motor (8) which is used to turn the rotor (9) between the operating positions.

25. Apparatus according to one of the claims 11-18 comprising a stator (7) comprising the air flow generator (4) and a bearing for the axis (71) of the rotor (9) which carries at least one, preferably up to five devices (1x, 1y, ...).

## Patentansprüche

1. Eine Vorrichtung (1) zum kontrollierten Transfer einer in einem Behälter (20; 201) gelagerten Flüssigkeit (21) durch eine Ausgabeöffnung (22) des Behälters (20; 201), wobei die Vorrichtung einen Behälter (20, 201) und ein erstes Kapillarmedium (3) enthält, dessen unterer Teil in eine nahe beim Boden (23) des Behälters (20; 201) gelegene Region ragt, worin Flüssigkeit aufgrund von Kapillarkräften durch die Öffnung (22) transferierbar ist, wobei die Lage des unteren Teiles (31) des ersten Kapillarmediums (3) bezüglich des Behälters (20; 201) fixiert ist und worin das erste Kapillarmedium (3) einen Docht beinhaltet, **dadurch gekennzeichnet, dass** der Hauptteil der seitlichen Oberfläche des Dochtes von einer für Flüssigkeit undurchlässigen Hülse umgeben ist, so dass die Oberfläche des Dochtes (3) an seinem unteren Teil (31) für Flüssigkeit (21) durchlässig und ansonsten für Flüssigkeit (21) undurchlässig ist.

2. Vorrichtung nach Anspruch 1, worin die Schicht (13) zusätzlich dazu dient, die Lage des ersten Kapillarmediums (3) bezüglich des Behälters (22; 201) zu stabilisieren.

3. Vorrichtung nach Anspruch 1, worin das erste Kapillarmedium (3) eine Anzahl an Kapillaren beinhaltet.

4. Vorrichtung nach einem der vorangegangenen Ansprüche, worin die Flüssigkeit, welche vom ersten Kapillarmedium transferiert worden ist, Luft oder einem Luftstrom ausgesetzt wird, welcher von einem Luftstromgenerator (4) zur Verdampfung erzeugt wird.

5. Eine Vorrichtung nach Anspruch 1, wobei der Behälter (20; 201) einen Halter (10) für ein Kapillarmedium (3) beinhaltet, welcher in eine nahe beim Boden (23) des Behälters (20; 201) gelegene Region ragt, wobei der Halter (10) eine Halterung (11) aufweist, welche konstruiert ist, um das Kapillarmedium (3) zu umfassen und derart in eine Öffnung (22) zu passen, dass Flüssigkeit nur durch die Öffnung (22) durch das Kapillarmedium (3) transferierbar ist, wobei der zur Halterung (11) angrenzende Halter (19) zudem die Hülse (13) umfasst, welche das Kapillarmedium (3) bis oder nahe bis zu dessen Ende (31) dicht umfasst, so dass die Vorrichtung (1) zwischen wenigstens einer ersten Lage, in welcher Flüssigkeit (21) transferiert wird, während sie in Kontakt zum Ende des Kapillarmediums (3) steht, und wenigstens einer zweiten Lage, in welcher das Ende (14) der Hülse (13) aus der Flüssigkeit (21) ragt, wodurch ein Kontakt der Flüssigkeit (21) mit dem Kapillarmedium (3) verhindert wird, gedreht werden kann.

6. Vorrichtung (1) nach Anspruch 5, worin das Kapillarmedium (3), welches aus der Halterung (11) ragt, mit einem zweiten Kapillarmedium (5) wie einer Gaze oder einem verlängerten Teil des ersten Kapillarmediums (3) verbunden ist, von welchem die transferierte Flüssigkeit (21) der Luft oder dem Luftstrom ausgesetzt wird, welcher von einem Luftstromgenerator (4) zur Verdampfung erzeugt wird.

7. Vorrichtung (1) nach einem der vorangehenden Ansprüche, worin der Behälter (201) mit wenigstens einem Reservoir (204a, 204b) ausgestattet ist, welches geeignet ist, die Flüssigkeit aufzunehmen, wenn der Behälter (201) in eine dritte, ungefähr horizontale Lage gedreht wird.

8. Vorrichtung (1) nach einem der vorhergehenden Ansprüche, worin die Öffnung (22) mit einer Abdeckung (25) verschlossen werden kann, welche eine Öffnung (26) beinhaltet, die geeignet ist, das Kapillarmedium (3) einzusetzen.

9. Vorrichtung (1) nach einem der Ansprüche 5 bis 8, worin die Halterung (11) einen Flansch (12) beinhaltet, welcher über die Kanten der Öffnung (22) ragt.

10. Vorrichtung (1) nach einem der vorhergehenden Ansprüche, worin die Flüssigkeit (21) ein Parfum, ein pharmazeutisches Agens, ein Maskierungsagens, ein Insektizid oder eine organoleptische Substanz ist.

11. Vorrichtung nach einem der Ansprüche 5 bis 10, worin das Kapillarmedium (3) ein Docht ist, wobei der Docht aus Web- oder Vliesstoff und/oder aus porösem Material besteht.

12. Vorrichtung (101; 102) nach einem der vorhergehenden Ansprüche beinhaltend einen ringförmigen Behälter (2000; 2001) mit wenigstens einem Reservoir (102a; 102b; 102c), welche mit einem Halter (10; 10a; 10b; 10c) für den Transfer einer Flüssigkeit (21; 21a; 21b; 21c) durch ein erstes Kapillarmedium (3) zu einem zweiten Kapillarmedium (5) ausgestattet ist, welches vom ringförmigen Behälter (2000; 2001) umfasst wird.

13. Vorrichtung (102) nach Anspruch 12, worin der Behälter (2001), welcher zwei oder mehrere Reservoirs (102a; 102b; 102c) enthält, aus einem Stück gebildet ist oder aus miteinander verbindbaren Segmenten (102a') besteht, die ein Reservoir (102a) enthalten.

14. Vorrichtung (1; 101; 102) nach einem der Ansprüche 5 bis 13, worin der Halter (10') im Behälter (29; 201, 2000, 2001) beziehungsweise im Segment (102a') eingebaut ist.

15. Gerät zur Verteilung wenigstens einer Flüssigkeit (21), beinhaltend wenigstens eine Vorrichtung (1x, 1y, 1z; 101; 102) nach einem der vorangehenden Ansprüche zum kontrollierbaren Transfer von Flüssigkeit (21) durch eine Ausgabeöffnung (22) eines Behälters (20; 201; 2000; 2001) an die Luft, wobei besagte Vorrichtung (1x, 1y, 1z; 101; 102) zwischen wenigstens einer ersten Lage, in welcher Flüssigkeit (21) transferiert wird, und einer zweiten Lage, in welcher der Transfer von Flüssigkeit verhindert wird, drehbar montiert ist.

16. Gerät nach Anspruch 15, worin die Flüssigkeit (21) über ein zweites Kapillarmedium (5), welches mit dem ersten Kapillarmedium (3) verbunden ist, an die Luft transferiert wird.

17. Gerät nach Anspruch 15 oder 16 mit einem Rotor (9), welcher um wenigstens eine erste Achse (w) gedreht werden kann, und mit wenigstens zwei Vorrichtungen (1x, 1y), welche in einer Ebene senkrecht zur ersten Achse (w) installiert sind, mit einem Abstand von 90°, 120° oder 180° von benachbarten Vorrichtungen, wobei in einer ersten Lage des Rotors (9) Flüssigkeit (21) nur in eine erste Vorrichtung (1x) transferiert wird, und dass in einer zweiten Lage des Rotors (9) Flüssigkeit (21) nur in eine zweite Vorrichtung (1y) transferiert wird.

18. Gerät nach Anspruch 15, 16 oder 17, wobei der Rotor (9) drehbar um die erste Achse (w) und eine zweite Achse (v) ist, und mit wenigstens vier Vorrichtungen, welche am Rotor (9) in ungefähr gleichen Abständen von einander auf einer imaginären Sphäre installiert sind.

19. Gerät nach einem der Ansprüche 15 bis 18, wobei die Ausgabeöffnung (22) und/oder das zweite Kapillarmedium (5) der installierten Vorrichtungen (1, 1x, 1y, 1z) zur ersten Achse (w) und/oder zum Schneidepunkt der ersten und der zweiten Achse (v, w) hin ausgerichtet ist.

20. Gerät nach einem der Ansprüche 15 bis 19 mit einer Vorrichtung (101), welcher einen ringförmigen Behälter (2000; 2001) mit wenigstens einem Reservoir (102a; 102b; 102c) beinhaltet, welcher mit einem Halter (10; 10a; 10b; 10c) für den Transfer einer Flüssigkeit (21; 21a; 21b; 21c) durch ein erstes Kapillarmedium (3) zu einem zweiten Kapillarmedium (5) ausgestattet ist, welcher vom ringförmigen Behälter (2000; 2001) umfasst wird.

21. Gerät nach einem der Ansprüche 15 bis 20, beinhaltend einen Luftstromgenerator (4) wie einen Ventilator, welcher einen Luftstrom wenigstens zur Abgabeöffnung (22) oder zum zweiten Kapillarmedium (5) der Vorrichtung (1, 1x, 1y, 1z) erzeugt, deren erstes Kapillarmedium (3) Flüssigkeit (21) transferiert.

22. Gerät nach Anspruch 21, welches weiter einen Kanal (93) enthält, um den Luftstrom vom Luftstromgenerator (4) zur Abgabeöffnung (22) oder zum zweiten Kapillarmedium hinzuführen.

23. Gerät nach einem der Ansprüche 15 bis 22, mit einer Steuerungseinheit (6) beinhaltend einen Schalter (61) zur Betätigung des Luftstromgenerators (4) und/oder beinhaltend einen Sensor (62) zur Ermittlung der Lage des Rotors (9), wobei die Steuerungseinheit (6) abhängig von der Lage des Rotors (9) den Luftstromgenerator (4) zu betätigen und/oder den Luftstrom zu kontrollieren vermag.

24. Gerät nach Anspruch 23, wobei die Steuerungseinheit (6) programmierbar ist und einen Motor (8) zu kontrollieren vermag, welcher verwendet wird, um den Rotor (9) zwischen den Betriebslagen zu drehen.

25. Gerät nach einem der Ansprüche 1 bis 18 beinhaltend einen Stator (7), welcher den Luftstromgenerator (4) und ein Lager für die Achse (71) des Rotors (9) enthält, welcher wenigstens eine, bevorzugt bis zu fünf Vorrichtungen (1x, 1y, ...) trägt.

## Revendications

1. Dispositif (1) pour le transfert contrôlable d'un liquide (21) stocké dans un conteneur (20 ; 201) à travers une ouverture de sortie (22) dudit conteneur (20 ; 201), ledit dispositif comportant un conteneur (20 ; 201) et un premier milieu capillaire (3) dont une partie inférieure s'étend dans une zone proche du fond (23) du conteneur (20 ; 201), du liquide pouvant être transféré à travers l'ouverture (22) du fait de l'action capillaire, de sorte que la position de la partie inférieure (31) du premier milieu capillaire (3) par rapport au conteneur (20 ; 201) est fixe, et le premier milieu capillaire (3) comportant une mèche, **caractérisé en ce que** la majeur partie des surfaces latérales de la mèche sont entourées par un manchon (13) imperméable au liquide, de sorte que la surface de la mèche (3) est perméable au liquide (21) au niveau de sa partie inférieure (31) , et est sinon imperméable au liquide (21).

2. Dispositif selon la revendication 1, dans lequel le manchon (13) sert de plus à stabiliser la position du premier milieu capillaire (3) par rapport au conteneur (20 ; 201).

3. Dispositif selon la revendication 1, dans lequel le premier milieu capillaire (3) comporte plusieurs capillaires.

4. Dispositif selon l'une quelconque des revendications précédentes, dans lequel le liquide transféré par le premier milieu capillaire est exposé à l'air ou à un écoulement d'air généré par un générateur d'écoulement d'air (4) pour évaporation.

5. Dispositif (1) selon la revendication 1, dans lequel le conteneur (20 ; 201) comporte un support (10) destiné à un milieu capillaire (3) qui s'étend dans une zone proche du fond (23) du conteneur (20 ; 201), ledit support (10) comportant un raccord (11) étant conçu pour enserrer le milieu capillaire (3) et pour se raccorder dans l'ouverture (22) d'une manière telle qu'un liquide peut seulement être transféré à travers l'ouverture (22) par le milieu capillaire (3), dans lequel le support (10), adjacent au raccord (11) , comporte de plus ledit manchon (13) qui enserre de manière serrée le milieu capillaire (3) jusqu'à son extrémité (31) ou à proximité de celle-ci, de sorte que le dispositif (1) peut être tourné entre au moins une première position dans laquelle le liquide (21) est transféré, tout en étant en contact avec l'extrémité (31) du milieu capillaire (3), et au moins une deuxième position dans laquelle l'extrémité (14) du manchon (13) s'étend à l'extérieur du liquide (21), en empêchant ainsi un contact du liquide (21) avec le milieu capillaire (3).

6. Dispositif (1) selon la revendication 5, dans lequel le milieu capillaire (3), qui s'étend à l'extérieur du raccord (11), est connecté à un second milieu capillaire (5) telle qu'une gaze ou une partie étendue du premier milieu capillaire (3), à partir de laquelle le liquide transféré (21) est exposé à l'air ou à l'écoulement d'air généré par un générateur d'écoulement d'air (4), et est évaporé.

7. Dispositif (1) selon l'une quelconque des revendications précédentes, dans lequel le conteneur (201) est muni d'au moins un réservoir (204a, 204b) qui est adapté pour recevoir le liquide (21) lorsque le conteneur (201) est tourné dans une troisième position approximativement horizontale.

8. Dispositif (1) selon l'une quelconque des revendications précédentes, dans lequel l'ouverture (22) peut être fermée par un couvercle (25) comportant une ouverture (26) adaptée pour insertion du milieu capillaire (3).

9. Dispositif (1) selon l'une quelconque des revendications 5 à 8, dans lequel le raccord (11) comporte un rebord (12) qui s'étend au-dessus des bords de l'ouverture (22).

10. Dispositif (1) selon l'une quelconque des revendications précédentes, dans lequel le liquide (21) est un parfum, un agent pharmaceutique, un agent de masquage, un insecticide ou une substance organoleptique.

11. Dispositif (1) selon l'une quelconque des revendications 5 à 10, dans lequel le milieu capillaire (3) est une mèche, la mèche étant constituée d'un tissu tissé ou non-tissé et/ou d'un matériau poreux.

12. Dispositif (101 ; 102) selon l'une quelconque des revendications précédentes, comportant un conteneur annulaire (2000 ; 2001) muni d'au moins un réservoir (102a ; 102b ; 102c) étant équipé d'un support (10 ; 10a ; 10b ; 10c) pour le transfert d'un liquide (21 ; 21a ; 21b ; 21c) à travers un premier milieu capillaire (3) vers un second milieu capillaire (5) qui est enserré par le conteneur annulaire (2000 ; 2001).

13. Dispositif (102) selon la revendication 12, dans lequel le conteneur (2001), qui comporte deux ou plus de deux réservoirs (102a ; 102b ; 102c), est moulé en une seule pièce, ou est constitué de segments pouvant être connectés mutuellement (102a') comportant un réservoir (102a).

14. Dispositif (1 ; 101 ; 102) selon l'une quelconque des revendications 5 à 13, dans lequel le support (10') est incorporé dans le conteneur (20 ; 201, 2000, 2001) respectif du segment (102a').

15. Dispositif pour distribuer au moins un liquide (21), comportant au moins un dispositif (1x, 1y, 1z ; 101 ; 102) selon l'une quelconque des revendications précédentes, pour le transfert contrôlable d'un liquide (21) à travers une ouverture de sortie (22) d'un conteneur (20 ; 201 ; 2000 ; 2001) vers l'air, ledit dispositif (1x 1y, 1z; 101 ; 102) étant monté de manière rotative entre au moins une première position dans laquelle le liquide (21) est transféré, et une seconde position dans laquelle un transfert du liquide (21) est empêché.

16. Dispositif selon la revendication 15, dans lequel le liquide (21) est transféré vers l'air via un second milieu capillaire (5) connecté au premier milieu capillaire (3).

17. Dispositif selon la revendication 15 ou 16, muni d'un rotor (9) qui peut être tourné autour d'au moins un premier axe (w), et ayant au moins deux dispositifs (1x, 1y) installés dans un plan perpendiculaire au premier axe (w), avec un espacement de 90°, 120° ou 180° de dispositifs adjacents, dans une première position du rotor (9), le liquide (21) étant transféré uniquement dans un premier dispositif (1x), et dans lequel dans la seconde position du rotor (9), le liquide (21) est uniquement transféré dans un second dispositif (ly).

18. Dispositif selon la revendication 15, 16 ou 17, le rotor (9) pouvant tourner autour du premier axe (w) et d'un second axe (v), et étant muni d'au moins quatre dispositifs installés sur le rotor (9) à des distances approximativement égales les uns des autres sur une sphère imaginaire.

19. Dispositif selon l'une quelconque des revendications 15 à 18, l'ouverture de sortie (22) et/ou le second milieu capillaire (5) des dispositifs installés (1, 1x, 1y, 1z) étant orienté(e) vers le premier axe (w) et/ou le point de recoupement des premier et second axes (v, w).

20. Dispositif selon l'une quelconque des revendications 15 à 19, muni d'un dispositif (101) comportant un conteneur annulaire (2000 ; 2001) muni d'au moins un réservoir (102a ; 102b ; 102c) équipé d'un support (10 ; 10a ; 10b ; 10c) pour le transfert d'un liquide (21 ; 21a ; 21b ; 21c) à travers un premier milieu capillaire (3) vers un second milieu capillaire (5) qui est enserré par le conteneur annulaire (2000 ; 2001).

21. Dispositif selon l'une quelconque des revendications 15 à 20, comportant un générateur d'écoulement d'air (4), tel qu'un ventilateur, qui génère un écoulement d'air vers au moins l'ouverture de sortie (22) ou le second milieu capillaire (5) du dispositif (1, 1x, 1y, 1z) dent le premier milieu capillaire (3) transfère le liquide (21) .

22. Dispositif selon la revendication 21, comportant de plus un canal (93) pour guider l'écoulement d'air à partir du générateur d'écoulement d'air (4) vexa ladite ouverture de sortie (22) ou ledit second milieu capillaire (5).

23. Dispositif selon l'une quelconque des revendications 15 à 22, muni d'une unité de commande (6) comportant un commutateur (61) pour actionner le générateur d'écoulement d'air (4) et/ou comportant un capteur (62) pour détecter la position du rotor (9), ladite unité de commande (6) pouvant actionner le générateur d'écoulement d'air (4) et/ou commander l'écoulement d'air selon la position du rotor (9).

24. Dispositif selon la revendication 23, ladite unité de commande (6) pouvant être programmée et étant capable de commander un moteur (8) qui est utilisé pour faire tourner le rotor (9) entre les positions de fonctionnement.

25. Dispositif selon l'une quelconque des revendications 11 à 18, comportant un stator (7) comportant le générateur d'écoulement d'air (4) et un palier pour l'axe (71) du rotor (9) qui transporte au moine un, et de préférence jusqu'à cinq dispositifs (1x, 1y, ...).
